# EUROPÄISCHE PATENTANMELDUNG

(11) **EP 3 698 723 A1**
(43) Veröffentlichungstag der Anmeldung: **26.08.2020**
(21) Anmeldenummer: 19158345.9
(22) Anmeldetag: 20.02.2019
(51) Int. Cl.: A61B 17/00, A61M 5/19

(54) **APPLIKATOR FÜR EINEN ZWEI-KOMPONENTEN GEWEBEKLEBER**

(71) Anmelder: Adhesys Medical GmbH, 52074 Aachen (DE)
(72) Erfinder: LOUGH, Hayley A., Boston, MA, 02199 (US); HAYES, Matthew, Boston. MA. 02127 (US); DORSCHEID, Ralf, 52134 Herzogenrath (DE)
(74) Vertreter: Davepon, Björn

(57) **Zusammenfassung**

Die vorliegende Erfindung betrifft ein Dosiersystem (1) mit wenigstens zwei Kammern (2, 3) für ein wenigstens zwei Komponenten umfassendes Gewebekleber-System, dadurch gekennzeichnet, dass die erste Kammer (2) als Hohlzylinder (H) ausgebildet ist, der an einer Stirnseite (2a) verschlossen und an seiner gegenüberliegenden Stirnseite (2b) offen ausgebildet ist und die zweite Kammer (3) in einem Vollzylinder (V) als Ringspalt (4) ausgebildet ist und sich der Ringspalt (4) von einer dem Hohlzylinder (H) zugewandten Stirnseite (3a) bis zu einer an dem gegenüberliegenden Ende (3b) des Vollzylinders (V) angeordneten Mischkammer (5) erstreckt und der Hohlzylinder (H) auf der Seite seiner offenen Stirnseite (2b) in dem Ringspalt (4) des Vollzylinders (V) in Längsrichtung (L) verschiebbar angeordnet ist, wobei in dem Vollzylinder (V) wenigstens ein Zuführungskanal (6) ausgebildet ist, der sich innenseitig des Ringspaltes (4) von der dem Hohlzylinder (H) zugewandten Stirnseite (3a) des Vollzylinders (V) bis in die Mischkammer (5) erstreckt, wobei in der Mischkammer (5) ein Mischrohr (7) vorgesehen ist, welches aus dem Vollzylinder (V) seitlich herausragt und im Wesentlichen quer zur Längsausdehnung des Vollzylinders (V) verschiebbar angeordnet ist und das in einer ersten Position das Innere des Mischrohres (7) gegenüber dem Zuführungskanal (6) und dem Ringspalt (4) verschließt und in einer zweiten Position freigibt. Die Erfindung betrifft ferner ein Kit (20) aus einem Dosiersystem (1), welches mit einem wenigstens zwei Komponenten umfassendes Gewebekleber-System befüllt ist sowie ein Verfahren zum Verschließen einer Wunde mithilfe eines solchen Kits (20).

## Beschreibung

Die vorliegende Erfindung betrifft ein Dosiersystem mit wenigstens zwei Kammern für ein wenigstens zwei Komponenten umfassendes Gewebekleber-System. Die Erfindung betrifft ferner ein Kit aus einem Dosiersystem, welches mit einem wenigstens zwei Komponenten umfassendes Gewebekleber-System befüllt ist sowie ein Verfahren zum Verschließen einer Wunde mithilfe eines solchen Kits.

Im Stand der Technik sind Zweikomponenten-Gewebekleber-Systeme (2K-Gewebekleber-Systeme) sowie entsprechende Dosiersysteme mit zwei Kammern beschrieben. So offenbart die EP 2 173 782 B1 ein 2-Kammer-Dosiersystem welches mit einem 2K-Gewebekleber-System befüllt ist, von denen die eine Komponente ein isocyanatfunktionelles Präpolymer aus einem Polyisocyanat und einem Polyol und die zweite Komponente ein aminofunktioneller Asparaginsäureester ist. Solche 2-Kammer-Dosiersysteme werden üblicherweise als medizinische Spritze ausgestaltet mit zwei parallel zueinander angeordneten Zylindern, die synchron ausgepresst werden, wobei die zwei Komponenten am Ausgang der Spritze in einer Leitung zusammengeführt und über einen mit einem Statikmischer versehenen Applikator auf die gewünschte Stelle aufgetragen werden können.

Bei dieser Lösung kann es zum Teil als nachteilig empfunden werden, dass die Bedienung nur von Fachpersonal durchgeführt werden kann. Typischerweise werden hierfür auch beide Hände benötigt. Zudem sind solche spritzenartigen 2-Kammer-Dosiersysteme mit parallel zueinander angeordneten Zylindern mechanisch nicht sonderlich stabil und beispielsweise empfindlich gegen Verbiegen, was insbesondere durch die konstruktionsbedingte längliche und zugleich dünne Form begünstigt wird, da die Kolben der Spritze wegen der gefüllten Spritzenzylinder in der Regel voll ausgezogen sind.

Die Aufgabe der vorliegenden Erfindung bestand somit darin, ein Dosiersystem mit wenigstens zwei Kammern für ein wenigstens zwei Komponenten umfassendes Gewebekleber-System anzugeben, welches auch von nicht medizinisch vorgebildeten Personen bedient werden kann, vorzugsweise mit einer Hand, damit beispielsweise Verletzungen am eigenen Arm oder der eigenen Hand ebenfalls behandelt werden können. Vorzugsweise soll das Dosiersystem mechanisch stabiler als die bislang bekannten spritzenartigen 2-Kammer-Dosiersysteme aufgebaut sein.

Diese Aufgabe wird erfindungsgemäß gelöst durch ein Dosiersystem mit wenigstens zwei Kammern für ein wenigstens zwei Komponenten umfassendes Gewebekleber-System, welches dadurch gekennzeichnet ist, dass die erste Kammer als Hohlzylinder ausgebildet ist, der an einer Stirnseite verschlossen und an seiner gegenüberliegenden Stirnseite offen ausgebildet ist und die zweite Kammer in einem Vollzylinder als Ringspalt ausgebildet ist und sich der Ringspalt von einer dem Hohlzylinder zugewandten Stirnseite bis zu einer an dem gegenüberliegenden Ende des Vollzylinders angeordneten Mischkammer erstreckt und der Hohlzylinder auf der Seite seiner offenen Stirnseite in dem Ringspalt des Vollzylinders in Längsrichtung verschiebbar angeordnet ist, wobei in dem Vollzylinder wenigstens ein Zuführungskanal ausgebildet ist, der sich innenseitig des Ringspaltes von der dem Hohlzylinder zugewandten Stirnseite des Vollzylinders bis in die Mischkammer erstreckt, wobei in der Mischkammer ein Mischrohr vorgesehen ist, welches aus dem Vollzylinder seitlich herausragt und im Wesentlichen quer zur Längsausdehnung des Vollzylinders verschiebbar angeordnet ist und das in einer ersten Position das Innere des Mischrohres gegenüber dem Zuführungskanal und dem Ringspalt verschließt und in einer zweiten Position freigibt.

Der Erfindung liegt die Erkenntnis zugrunde, dass durch die ineinander verschiebbare Anordnung und Ausgestaltung der Kammern das Dosiersystem deutlich kompakter als die bislang bekannten spritzenartigen 2-Kammer-Dosiersysteme ausgelegt werden kann. Hierdurch kann das Dosiersystem beispielsweise eine rechteckige Geometrie besitzen die deutlich biegesteifer als eine befüllte 2-Kammer-Spritze ist. Dadurch wird es auch möglich, das Dosiersystem handergonomischer zu gestalten, sodass es auch von im Umgang hiermit un- oder wenig geübten Personen in einer Hand zusammengedrückt und das 2K-Gewebekleber-System auf diese Weise ausgepresst werden kann.

Bevorzugt ist das erfindungsgemäße Dosiersystem mit zwei Kammern für ein zwei Komponenten umfassendes Gewebekleber-System ausgebildet.

Das erfindungsgemäße Dosiersystem weist unter anderem einen Hohlzylinder und einen Vollzylinder auf. Unter einem Zylinder wird im Sinne der vorliegenden Erfindung nicht nur ein einfacher Kreiszylinder sondern auch Zylinder mit ovaler, (viel)eckiger, wie beispielsweise viereckiger, hexagonaler oder prismischer Querschnittsfläche, ellipsoider oder trapezoider Querschnittsfläche verstanden. Dabei sind der Hohlzylinder und der Vollzylinder so aufeinander abgestimmt, dass sie ineinander verschoben werden können. Die Längsausdehnung des Hohlzylinders ist bevorzugt derart gewählt, dass die Höhe der Innenwand weitestgehend identisch mit der Tiefe des Ringspalts ist.

Der Vollzylinder kann monolithisch beziehungsweise aus einem Vollmaterial aufgebaut sein oder aber auch Hohlräume zur Materialeinsparung aufweisen, die jedoch nicht der Befüllung mit Komponenten des 2K-Gewebekleber-Systems dienen.

Der Vollzylinder ist vorzugsweise aus zwei Elementen ausgebaut, wodurch das Zusammenfügen mit dem Hohlzylinder erleichtert wird. Dabei beherbergt das erste Element im wesentlichen den Ringspalt während das zweite Element über den Hohlzylinder geschoben und beispielsweise über eine Rastverbindung mit dem ersten Element fest verbunden werden kann, sodass Hohl- und Vollzylinder eine Einheit bilden. Der Vollzylinder kann im Inneren gegenüber den Klebstoffkomponenten abgeschlossene Hohlräume zur Materialeinsparung aufweisen. Diese Hohlräume werden beim Befüllen des Ringspaltes des Vollzylinders somit nicht mit Klebstoffkomponenten gefüllt.

Der Hohlzylinder und der Vollzylinder sind bevorzugt aus einem biegesteifen Material gefertigt, insbesondere aus einem Kunststoffmaterial dessen Polymermatrix vorzugsweise ausgewählt ist aus Polyolefinen, bevorzugt Polyethylen und Polypropylen, ABS-Kunststoff, Polycarbonat, Polyester, bevorzugt Polyethylenterephthalat, Polymethyl(meth)acrylat, Polyvinylchlorid, (2K) Epoxydharzen, Polystyrol sowie Mischungen und Mischpolymerisaten von diesen. Die Herstellung des Hohlzylinder und des Vollzylinders kann auf alle dem Fachmann zur Formgebung der oben genannten Materialien bekannten Verfahren erfolgen, wie beispielsweise Spritzgießen.

Bei dem erfindungsgemäßen Dosiersystem kann im Bereich der Stoßkante der offenen Stirnseite des Hohlzylinders eine Anschlageinrichtung ausgebildet sein, die mit einer an der dem Hohlzylinder zugewandten Stirnseite des Vollzylinders ausgebildeten korrespondierenden Anschlageinrichtung in der Weise zusammenwirkt, dass der Hohlzylinder und der Vollzylinder nur bis zum gegenseitigen Kontakt der Anschlageinrichtungen auseinander gezogen werden können. Hierdurch wird beim Befüllen des Dosiersystems sichergestellt, dass die Kammern nur bis zum Erreichen des Anschlags gefüllt werden. Zudem wird durch die Anschlageinrichtung ein unbeabsichtigtes Auseinanderziehen des Dosiersystems verhindert.

Der Hohlzylinder und der Vollzylinder können ferner mit einer Sicherungseinrichtung versehen sein, die den Hohlzylinder und den Vollzylinder gegen ein unbeabsichtigtes gegeneinander Verschieben sichert. Hierfür kann an einer der beiden Zylinder eine Kunststoffnase vorgesehen sein, die mit einer entsprechenden Aussparung am anderen Zylinder korrespondiert und die bei beabsichtigter Benutzung durch erhöhe Krafteinwirkung abbricht und damit die Zylinder freigibt, sodass sie gegeneinander verschoben werden können.

Zweckmäßigerweise ist die Anschlageinrichtung am Hohlzylinder als umlaufender Ringwulst ausgebildet, der mit einer am Vollzylinder innenseitig umlaufenden Anschlagfläche als korrespondierende Anschlageinrichtung zusammenwirkt. Dabei kann weiterhin an der Ringwulst ein umlaufendes Dichtelement vorgesehen sein, welches mit wenigstens einer, vorzugsweise mit beiden, der Längsflächen des Ringspaltes dichtend zusammenwirkt.

Die Volumina der beiden Kammern können über weite Bereiche frei gewählt werden und werden zweckmäßigerweise so aufeinander abgestimmt, dass sie dasselbe Verhältnis aufweisen wie das, in dem die Komponenten des zwei Komponenten umfassenden Gewebekleber-Systems miteinander gemischt werden müssen. Bevorzugt liegt das Volumenverhältnis der Kammern bei 1 : 10 bis 10 : 1, bevorzugt bei 5 : 1 bis 1 : 5.

Nach einer bevorzugten Ausführungsform des erfindungsgemäßen Dosiersystems ist das Volumen der ersten Kammer größer als dasjenige der zweiten Kammer, wobei das Volumen der ersten Kammer zum Volumen der zweiten Kammer im Verhältnis 5 : 1 bis 3 : 1 steht, bevorzugt 4,5 : 1 bis 3,5 : 1.

Der Hohlzylinder und der Vollzylinder können wie vorstehend bereits ausgeführt eine kreisrunde, ovale, (viel)eckige, ellipsoide oder trapezoide Querschnittsfläche aufweisen, insbesondere eine rechteckige Querschnittsfläche mit abgerundeten Kanten, wobei das Verhältnis der langen zur kurzen Seite bevorzugt wenigstens 3 : 1, weiter bevorzugt wenigstens 4 : 1 beträgt.

Zur Verbesserung der Handhabung und Griffigkeit des erfindungsgemäßen Dosiersystems kann der Hohlzylinder und / oder der Vollzylinder auf Außenseite mit einer in Längsrichtung verlaufender Profilierung versehen ist, insbesondere einer Riffelung.

In bevorzugter Weiterbildung des erfindungsgemäßen Dosiersystems sind innerhalb des Mischrohres Mischelemente angeordnet sind, wobei das Mischrohr insbesondere als Statikmischer ausgebildet ist. Hierdurch kann eine ausreichende Vermischung der Reaktionspartner des 2K- oder Mehrkomponenten-Gewebekleber-Systems bis zum verlassen des Mischrohres sichergestellt werden. An dem Mischrohr kann ferner eine Rasteinrichtung vorgesehen sein, die das Mischrohr in der ersten Position gegen unbeabsichtigtes Herausziehen des Mischrohrs fixiert. An dem Mischrohr kann eine Rückstellsperre vorgesehen sein, welche derart ausgebildet ist, dass sie das Mischrohr nach dem Verschieben in die zweite Position fixiert und ein Zurückschieben verhindert, wobei die Rückstellsperre bevorzugt als Rastelement zum Beispiel (Flügel oder Widerhaken) ausgebildet ist. Der Flügel kann beispielsweise aus einem mechanisch vorgespannten Kunststofffolien-Abschnitt bestehen, welcher sich nach dem Herausziehen des Mischrohres durch seine Vorspannung vom Mischrohr abstellt und dieses hierdurch gegen ein Zurückschieben blockiert. Das Mischrohr kann weiterhin auf seiner aus dem Vollzylinder herausragenden Seite mit einem abnehmbaren Verschluss versehen sein.

Bei dem erfindungsgemäßen Dosiersystem kann ferner vorgesehen sein, dass das Mischrohr verdrehsicher in der Mischkammer geführt und an seinem nach innen weisenden Ende auf der dem Zuführungskanal zugewandten Seite mit einer oder mehreren Öffnungen versehen ist, durch die der Zuführungskanal und / oder der Ringspalt mit dem Inneren des Mischrohres verbunden werden kann wenn das Mischrohr in die zweite Position gebracht wird. Zu diesem Zweck kann in der Mischkammer eine Führungsnase oder eine Führungsfeder vorgesehen sein, die mit einer entsprechenden Führungsnut am Mischrohr korrespondiert. Auch die umgekehrte Anordnung von Führungsnase beziehungsweise Führungsfeder und Führungsnut sind möglich.

In bevorzugter Weiterbildung des erfindungsgemäßen Dosiersystems ist die Mischkammer innerhalb des Vollzylinders ausgebildet. Dies ist vorteilhaft, weil auf diese Weise kein zusätzliches Bauteil benötigt wird was den Fertigungsprozess vereinfacht und zudem die Gefahr von Leckagen reduziert. Die Mischkammer dient in erster Linie dazu, das Mischrohr aufzunehmen und zu führen, wohingegen das eigentliche Vermischen der Komponenten innerhalb des Mischrohres stattfindet.

Besonders bevorzugt ist es, dass in der Mischkammer zwischen der Einmündung des Ringspalts und der Außenöffnung der Mischkammer eine Ringspaltdichtung vorgesehen ist, die der Abdichtung zwischen der Mischkammer und der äußeren Oberfläche des Mischrohrs in Position 1 und Position 2 dient. Auf diese Weise kann verhindert werden, dass der Inhalt der Kammern am Mischrohr vorbei nach außen gelangen können. Unabhängig hiervon können weiter innenliegend zusätzliche Ringspaltdichtungen vorgesehen sein, beispielsweise eine zwischen dem Durchlass des Ringspalts und dem Zuführungskanal, um ein unbeabsichtigtes Vermischen der wenigstens zwei Komponenten außerhalb des Mischrohres in der Mischkammer zu verhindern. Eine weitere Ringspaltdichtung kann auf der dem Durchlass des Ringspalts abgewandten Seite des Zuführungskanals angeordnet sein. Hierdurch kann eine Leckage der im Hohlzylinder enthaltenen Komponente in die Mischkammer unterbunden werden.

Der Zuführungskanal verläuft zweckmäßigerweise im Wesentlichen parallel zum Ringspalt und ist von diesem insbesondere wenigstens 2 mm beabstandet, vorzugsweise wenigstens 3 mm.

Zur besseren Versiegelung und Schutz insbesondere gegen Licht, Sauerstoff- und Feuchtigkeitseinwirkung können Hohlzylinder und Vollzylinder gemeinsam in einen Beutel eingeschlossen sein, wobei der Beutel vorzugsweise im Bereich des herausragenden Mischrohres mit einer Schwächungslinie versehen ist, welche ein gezieltes Öffnen des Beutels am Mischrohr erlaubt. Der Beutel zeichnet sich im Wesentlichen dadurch aus, dass er eine aus einem Material oder einer Material Komposition besteht, welche Lichtdicht und/oder Feuchtigkeitsundurchlässig und ferner alterungsbeständig ist sowie eine langfristige sterile Barriere gewährleistet. Der Beutel kann beispielsweise aus einer Verbundfolie bestehen, wie einer Aluminiumfolie, welche ein- oder beidseitig mit einem Polymerfilm kaschiert ist, beispielsweise mit einem Polyesterfolie (PET) und/oder einer Folie aus Polyethylen (PE). Der Beutel kann zudem vakuumiert oder auch mit einem inerten Schutzgas befüllt ein, wie beispielsweise Stickstoff.

Ein weiterer Gegenstand der vorliegenden Erfindung betrifft ein Kit aus einem erfindungsgemäßen Dosiersystem und einem darin befindlichen zwei Komponenten umfassenden Gewebekleber-System, sowie optional eine Verfahrensanleitung zum Verschließen einer Wunde mithilfe des Kits, wobei das Gewebekleber-System bevorzugt wenigstens einen Asparaginsäureester als Härter mit optional wenigstens einem Füllstoff als erste Komponente und als zweite Komponente ein isocynatfunktionelles Präpolymer umfasst, wobei sich die erste Komponente besonders bevorzugt in der ersten Kammer und die zweite Komponente bevorzugt in der zweiten Kammer des Dosiersystems befindet. Besonders bevorzugt werden als 2K-Gewebekleber-System solche eingesetzt, wie sie in EP 2 173 782 B1, EP 2 182 018 B1, WO 2011/006606 A1, WO 2010/006714 A2,A3, EP 2 699 615 B1, WO 2013/104563 A1, WO 2012/107375 A1, WO 2013/092506 A1, EP 2 699 614 B1 oder WO 2013/092504 A1 offenbart sind.

Die vorliegende Erfindung betrifft ferner ein Verfahren zum Verschließen einer Wunde mithilfe eines erfindungsgemäßen Kits umfassend die Schritte:
a) Herausziehen des Mischrohrs bis zum Erreichen der zweiten Position;
b) Druckausübung auf den Hohlzylinder und den Vollzylinder relativ zueinander in Längsrichtung der Zylinder, wodurch die beiden Komponenten des Gewebekleber-Systems in Richtung der Mischkammer gefördert und beim Durchfließen des Mischrohres miteinander vermischt werden;
c) Optionales Verwerfen der wenigstens ersten 10 mm aus dem Mischrohr austretenden Gewebeklebers;
d) Auftragen des unter weiterer Druckausübung aus dem Mischrohr austretenden Gewebeklebers auf die Wunde;
e) Aushärtenlassen des Gewebeklebers.

Wenn das System in einem Folienbeutel eingeschlossen ist, kann dieser im Bereich des Mischrohres aufgerissen und das Mischrohr herausgezogen werden, gefolgt von den weiteren angegebenen Verfahrensschritten. Hierbei ist es also nicht erforderlich, den Folienbeutel zu entfernen.

Die Erfindung betrifft insbesondere die folgenden Ausführungsformen:
1. Dosiersystem 1 mit wenigstens zwei Kammern 2, 3 für ein wenigstens zwei Komponenten umfassendes Gewebekleber-System, dadurch gekennzeichnet, dass die erste Kammer 2 als Hohlzylinder H ausgebildet ist, der an einer Stirnseite 2a verschlossen und an seiner gegenüberliegenden Stirnseite 2b offen ausgebildet ist und
   die zweite Kammer 3 in einem Vollzylinder V als Ringspalt 4 ausgebildet ist und sich der Ringspalt 4 von einer dem Hohlzylinder H zugewandten Stirnseite 3a bis zu einer an dem gegenüberliegenden Ende 3b des Vollzylinders V angeordneten Mischkammer 5 erstreckt und der Hohlzylinder H auf der Seite seiner offenen Stirnseite 2b in dem Ringspalt 4 des Vollzylinders V in Längsrichtung L verschiebbar angeordnet ist,
   wobei in dem Vollzylinder V wenigstens ein Zuführungskanal 6 ausgebildet ist, der sich innenseitig des Ringspaltes 4 von der dem Hohlzylinder H zugewandten Stirnseite 3a des Vollzylinders V bis in die Mischkammer 5 erstreckt, wobei in der Mischkammer 5 ein Mischrohr 7 vorgesehen ist, welches aus dem Vollzylinder V seitlich herausragt und im Wesentlichen quer zur Längsausdehnung des Vollzylinders V verschiebbar angeordnet ist und das in einer ersten Position das Innere des Mischrohres 7 gegenüber dem Zuführungskanal 6 und dem Ringspalt 4 verschließt und in einer zweiten Position freigibt.
2. Dosiersystem nach Ausführungsform 1, dadurch gekennzeichnet, dass der Hohlzylinder H und der Vollzylinder V aus einem biegesteifen Material gefertigt sind, insbesondere aus einem Kunststoffmaterial dessen Polymermatrix vorzugsweise ausgewählt ist aus Polyolefinen, bevorzugt Polyethylen und Polypropylen, ABS-Kunststoff, Polycarbonat, Polyester, bevorzugt Polyethylenterephthalat, Polymethylmethacrylat, Polyvinylchlorid, Epoxyd-Harzen, Polystyrol sowie Mischungen und Mischpolymerisaten von diesen.
3. Dosiersystem nach Ausführungsform 1 oder 2, dadurch gekennzeichnet, dass im Bereich der Stoßkante der offenen Stirnseite 2b des Hohlzylinders H eine Anschlageinrichtung 8 ausgebildet ist, die mit einer an der dem Hohlzylinder H zugewandten Stirnseite 3a des Vollzylinders V ausgebildeten korrespondierenden Anschlageinrichtung 9 in der Weise zusammenwirkt, dass der Hohlzylinder H und der Vollzylinder V nur bis zum gegenseitigen Kontakt der Anschlageinrichtungen 8, 9 auseinander gezogen werden können.
4. Dosiersystem nach Ausführungsform 3, dadurch gekennzeichnet, dass die Anschlageinrichtung 8 am Hohlzylinder H als umlaufender Ringwulst ausgebildet ist, der mit einer am Vollzylinder V innenseitig umlaufenden Anschlagfläche als korrespondierende Anschlageinrichtung 9 zusammenwirkt.
5. Dosiersystem nach Ausführungsform 4, dadurch gekennzeichnet, dass an der Ringwulst ein umlaufendes Dichtelement 11 vorgesehen ist, welches mit wenigstens einer, vorzugsweise mit beiden, der Längsflächen des Ringspaltes 4 dichtend zusammenwirkt.
6. Dosiersystem nach einer der vorstehenden Ausführungsformen, dadurch gekennzeichnet, dass die Längsausdehnung des Hohlzylinders H derart gewählt ist, dass die Höhe der Innenwand weitestgehend identisch mit der Tiefe des Ringspalts 4 ist.
7. Dosiersystem nach einer der vorstehenden Ausführungsformen, dadurch gekennzeichnet, dass das Dosiersystem zwei Kammern 2, 3 aufweist und die Volumina der beiden Kammern 2, 3 in einem Verhältnis 1 : 10 bis 10 : 1 zueinander stehen, bevorzugt 5 : 1 bis 1 : 5.
8. Dosiersystem nach einer der vorstehenden Ausführungsformen, dadurch gekennzeichnet, dass das Volumen der ersten Kammer 2 zum Volumen der zweiten Kammer 3 im Verhältnis 5 : 1 bis 3 : 1 beträgt, bevorzugt 4,5 : 1 bis 3,5 : 1.
9. Dosiersystem nach einer der vorstehenden Ausführungsformen, dadurch gekennzeichnet, dass der Hohlzylinder H und der Vollzylinder V mit einer Sicherungseinrichtung versehen sind, die den Hohlzylinder H und den Vollzylinder V gegen ein unbeabsichtigtes gegeneinander Verschieben sichert.
10. Dosiersystem nach einer der vorstehenden Ausführungsformen, dadurch gekennzeichnet, dass der Hohlzylinder H und der Vollzylinder V eine kreisrunde, ovale, vieleckige, ellipsoide, trapezoide Querschnittsfläche aufweisen, insbesondere eine rechteckige Querschnittsfläche mit abgerundeten Kanten, wobei das Verhältnis der langen zur kurzen Seite bevorzugt wenigstens 3 : 1, weiter bevorzugt wenigstens 4 : 1 beträgt.
11. Dosiersystem nach einer der vorstehenden Ausführungsformen, dadurch gekennzeichnet, dass der Hohlzylinder H und / oder der Vollzylinder V auf Außenseite mit einer in Längsrichtung verlaufender Profilierung 12 versehen ist, insbesondere einer Riffelung.
12. Dosiersystem nach einer der vorstehenden Ausführungsformen, dadurch gekennzeichnet, dass innerhalb des Mischrohres 7 Mischelemente angeordnet sind, wobei das Mischrohr 7 insbesondere als Statikmischer ausgebildet ist.
13. Dosiersystem nach einer der vorstehenden Ausführungsformen, dadurch gekennzeichnet, dass an dem Mischrohr 7 eine Rasteinrichtung 12 vorgesehen ist, die das Mischrohr 7 in der ersten Position gegen unbeabsichtigtes Herausziehen des Mischrohrs 7 fixiert.
14. Dosiersystem nach einer der vorstehenden Ausführungsformen, dadurch gekennzeichnet, dass an dem Mischrohr 7 eine Rückstellsperre 13 vorgesehen ist, welche derart ausgebildet ist, dass sie das Mischrohr 7 nach dem Verschieben in die zweite Position fixiert und ein Zurückschieben verhindert, wobei die Rückstellsperre 13 bevorzugt als Rastelement, insbesondere als Flügel oder Widerhaken ausgebildet ist.
15. Dosiersystem nach einer der vorstehenden Ausführungsformen, dadurch gekennzeichnet, dass das Mischrohr 7 verdrehsicher in der Mischkammer 5 geführt und an seinem nach innen weisenden Ende auf der dem Zuführungskanal zugewandten Seite mit einer oder mehreren Öffnungen 14, 15 versehen ist, durch die der Zuführungskanal 6 und / oder der Ringspalt 4 mit dem Inneren des Mischrohres 7 verbunden werden kann wenn das Mischrohr 7 in die zweite Position gebracht wird.
16. Dosiersystem nach einer der vorstehenden Ausführungsformen, dadurch gekennzeichnet, dass das Mischrohr 7 auf seiner aus dem Vollzylinder V herausragenden Seite mit einem abnehmbaren Verschluss 16 versehen ist.
17. Dosiersystem nach einer der vorstehenden Ausführungsformen, dadurch gekennzeichnet, dass die Mischkammer 5 innerhalb des Vollzylinders V ausgebildet ist.
18. Dosiersystem nach einer der vorstehenden Ausführungsformen, dadurch gekennzeichnet, dass in der Mischkammer 5 zwischen der Einmündung des Ringspalts und der Außenöffnung der Mischkammer eine Ringspaltdichtung 17 vorgesehen ist, die der Abdichtung zwischen der Mischkammer 5 und der äußeren Oberfläche des Mischrohrs 7 in Position 1 und Position 2 dient.
19. Dosiersystem nach einer der vorstehenden Ausführungsformen, dadurch gekennzeichnet, dass der Zuführungskanal 6 im Wesentlichen parallel zum Ringspalt 4 verläuft und von diesem insbesondere wenigstens 2 mm beabstandet ist, vorzugsweise wenigstens 3 mm.
20. Dosiersystem nach einer der vorstehenden Ausführungsformen, dadurch gekennzeichnet, dass Hohlzylinder H und Vollzylinder V gemeinsam in einen Beutel 21 eingeschlossen sind, wobei der Beutel 21 vorzugsweise im Bereich des herausragenden Mischrohres 7 mit einer Schwächungslinie versehen ist, welche ein gezieltes Öffnen des Beutels 19 am Mischrohr 7 erlaubt.
21. Kit 20 aus einem Dosiersystem 1 nach einer der Ausführungsformen 1 bis 20, und einem darin befindlichen zwei Komponenten umfassenden Gewebekleber-System, sowie optional eine Verfahrensanleitung zum Verschließen einer Wunde mithilfe des Kits, wobei das Gewebekleber-System bevorzugt wenigstens einen Asparaginsäureester als Härter mit optional wenigstens einem Füllstoff als erste Komponente und als zweite Komponente ein isocynatfunktionelles Präpolymer umfasst, wobei sich die erste Komponente besonders bevorzugt in der ersten Kammer 2 und die zweite Komponente bevorzugt in der zweiten Kammer 3 des Dosiersystems befindet.
22. Verfahren zum Verschließen einer Wunde mithilfe eines Kits gemäß Ausführungsform 21 umfassend die Schritte:
   a) Herausziehen des Mischrohrs 7 bis zum Erreichen der zweiten Position;
   b) Druckausübung auf den Hohlzylinder H und den Vollzylinder V relativ zueinander in Längsrichtung der Zylinder H, V, wodurch die beiden Komponenten des Gewebekleber-Systems in Richtung der Mischkammer 5 gefördert und beim Durchfließen des Mischrohres 7 miteinander vermischt werden;
   c) Optionales Verwerfen der wenigstens ersten 10 mm aus dem Mischrohr 7 austretenden Gewebeklebers;
   d) Auftragen des unter weiterer Druckausübung aus dem Mischrohr 7 austretenden Gewebeklebers auf die Wunde;
   e) Aushärtenlassen des Gewebeklebers.

Die vorliegende Erfindung wird im Folgenden anhand eines in den Fign. 1 bis 6 gezeigten Ausführungsbeispiels näher erläutert. Darin zeigt
Fig. 1 Eine Ausführungsform einer erfindungsgemäßen Vorrichtung in seitlicher Draufsicht,
Fign. 2a) und b) Detailansichten der ersten Kammer der Vorrichtung aus Fig. 1 von schräg oben,
Fign. 3a) und b) Detailansichten der zweiten Kammer der Vorrichtung aus Fig. 1 von schräg oben,
Fig. 4 eine Schnittdarstellung der Vorrichtung aus Fig. 1 von schräg oben,
Fign. 5a) und b) Detailansichten des Mischrohres der Vorrichtung aus Fig. 1 in seitlicher Draufsicht und seitlicher Schnittdarstellung, sowie
Fign. 6a) und b) eine Ausführungsform eines erfindungsgemäßen Kits vor und nach dem Öffnen.

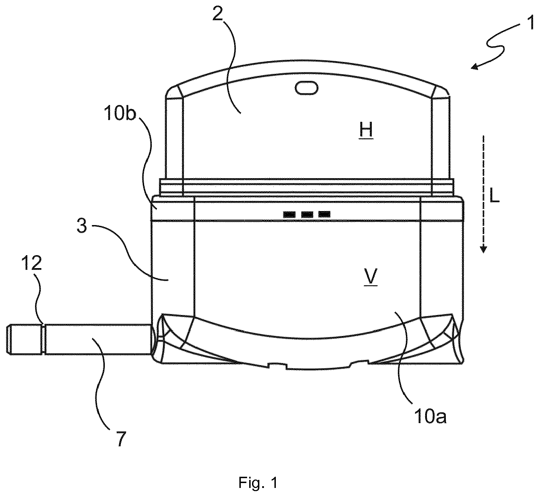

In Fig. 1 ist ein erfindungsgemäßes Dosiersystem 1 in seitlicher Draufsicht dargestellt. Das Dosiersystem 1 besteht aus zwei längsovalen Kammern 2, 3 für ein zwei Komponenten umfassendes Gewebekleber-System, beispielsweise ein solches basierend auf isocyanatfunktionellen Präpolymeren auf Basis von aliphatischen Polyisocyanaten und aminofunktionellen Asparaginsäureestern als eine Komponente und einem Polyol als zweite Komponente. Der Hohlzylinder H und der Vollzylinder V sind aus einem biegesteifen Material gefertigt, vorliegend aus Polypropylen.

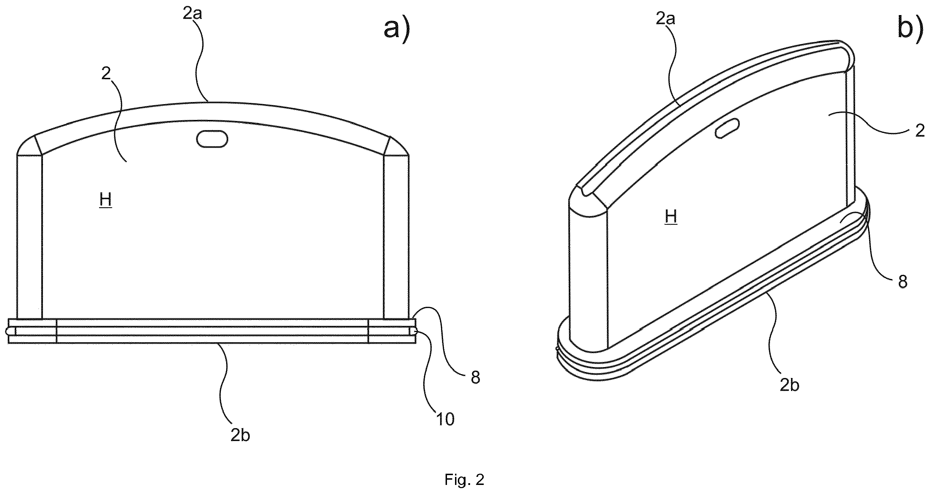

Die erste Kammer 2 ist als Hohlzylinder H ausgebildet ist, der an einer Stirnseite 2a verschlossen und an seiner gegenüberliegenden Stirnseite 2b offen ausgebildet ist, wodurch eine Kavität für die Aufnahme einer Komponente des Gewebekleber-Systems gebildet wird. Die erste Kammer 2 ist in den Fign 2a) und b) nochmals separat dargestellt.

Die zweite Kammer 3 ist in einem Vollzylinder V als ovaler Ringspalt 4 ausgebildet. In den Ringspalt 4 kann die zweite Komponente des Gewebekleber-Systems eingefüllt werden. Der Ringspalt 4 erstreckt sich von einer dem Hohlzylinder H zugewandten Stirnseite 3a bis zu einer an dem gegenüberliegenden Ende 3b des Vollzylinders V in diesem ausgebildeten Mischkammer 5, wobei der Hohlzylinder H auf der Seite seiner offenen Stirnseite 2b in dem Ringspalt 4 des Vollzylinders V in Längsrichtung L verschiebbar angeordnet ist.

In der Mischkammer 5 ist ein Mischrohr 7 vorgesehen, das vorliegend als Statikmischer ausgelegt ist. Das Mischrohr 7 ragt aus dem Vollzylinder V seitlich heraus und ist im Wesentlichen quer zur Längsausdehnung des Vollzylinders V verschiebbar angeordnet.

Die Volumina der beiden Kammern 2, 3 können dem Mischungsverhältnis des Klebstoffsystems entsprechend angepasst werden, wobei das Verhältnis der ersten Kammer 2 zur zweiten Kammer 3 vorliegend bei etwa 3 : 1 liegt.

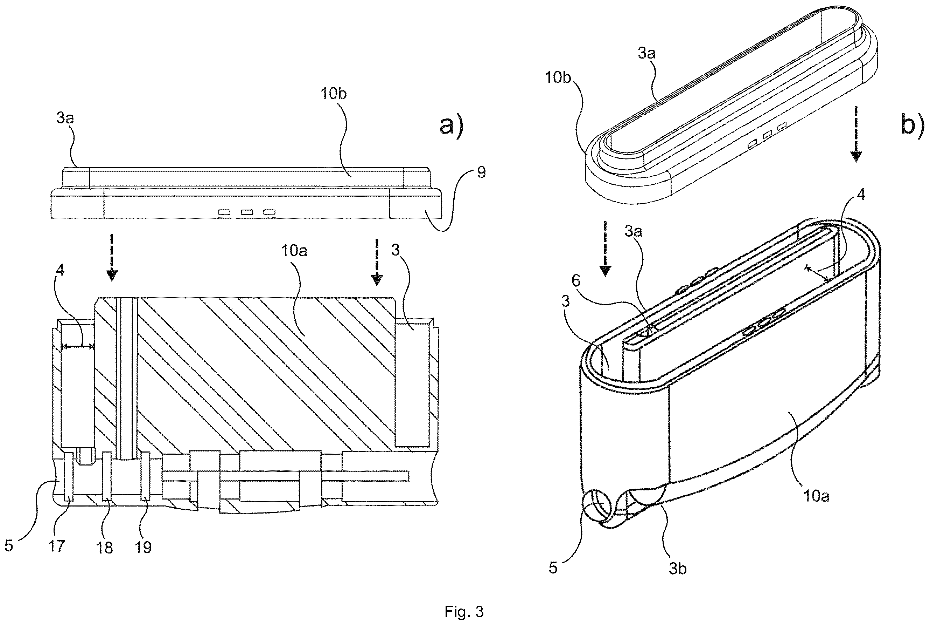

Wie in den Fign. 3a) und b) zu erkennen ist, ist die zweite Kammer 3 aus zwei Elementen 10a, 10b aufgebaut, die mittels einer Rasteinrichtung nach Zusammenfügen in der mit den gestrichelten Pfeilen angedeuteten Richtung fest und gasdicht miteinander verbunden werden können. Zum Zwecke der Abdichtung zwischen den zwei Elementen 10a, 10b ist eines von diesen mit einer umlaufenden Dichtung ausgestattet.

Die Aufteilung der zweiten Kammer 3 in die zwei Elemente 10a, 10b dient dem Zweck der einfacheren Befüllung der Kammern 2, 3 mit den Komponenten des Gewebekleber-Systems. So kann zunächst die erste Kammer 2 mit der einer Komponente des Gewebekleber-Systems befüllt und mit dem den Ringspalt 4 tragende Element 10a in der Weise zusammengesetzt werden, dass der umlaufende Randwulst der ersten Kammer 2 in den Ringspalt 4 eingeführt wird. Anschließend wird das ovale ringförmige zweite Element 10b über die erste Kammer 2 gestreift und mit dem Element 10a über die vorbeschriebene Rasteinrichtung verbunden, wodurch gleichzeitig die erste Kammer 2 verklemmt wird, sodass sie sich nicht mehr lösen kann. Anschließend wird der Ringspalt 4 der zweiten Kammer 3 mit der anderen Komponente des Gewebekleber-Systems befüllt. Dies erfolgt zweckmäßigerweise Vor dem Einführen des Mischrohres 7 über die für das Mischrohr 7 vorgesehene Öffnung der zweiten Kammer 3. Alternativ kann auch der Bodenbereich der Kammer 3 unterhalb der Mischkammer 5 abnehmbar ausgeführt sein, wodurch das Einfüllen in den Ringspalt 4 erleichtert wird.

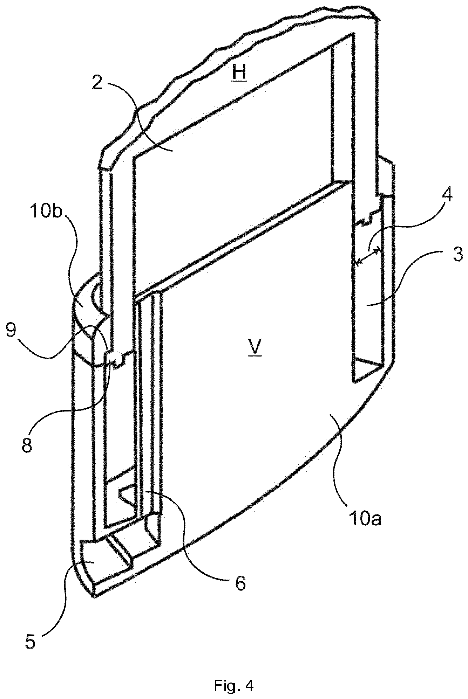

Im gefüllten und zusammengesetzten Zustand dient die Unterseite der umlaufenden Ringwulst der ersten Kammer 2 als Zylinderfläche und die Oberseite des Ringwulstes in Zusammenspiel mit der umlaufenden Unterkante als korrespondierende Anschlageinrichtungen 8, 9 des Hohlzylinders H und des Vollzylinders V, sodass der Hohlzylinder H und der Vollzylinder V nur bis zum gegenseitigen Kontakt der Anschlageinrichtungen 8, 9 auseinander gezogen werden können. Hierdurch wird ein unbeabsichtigtes Öffnen des Dosiersystems 1 verhindert. Damit beim Zusammendrücken des Dosiersystems die Klebstoffkomponenten möglichst vollständig genutzt werden können ist die Längsausdehnung des Hohlzylinders H derart gewählt, dass die Höhe der Innenwand weitestgehend identisch mit der Tiefe des Ringspalts 4 ist. Dies ist insbesondere aus der Schnittdarstellung in Fig. 4 zu erkennen.

In dem Vollzylinder V ist wenigstens ein Zuführungskanal 6 ausgebildet, der sich innenseitig des Ringspaltes 4 von der dem Hohlzylinder H zugewandten Stirnseite 3a des Vollzylinders V bis in die Mischkammer 5 erstreckt. Durch diesen Zuführungskanal 6 wird die in dem Hohlzylinder H enthaltene Gewebeklebstoffkomponente in Richtung der Mischkammer 5 gefördert, sobald die Kammern 2, 3 durch Zusammendrücken des Dosiersystems 1 ineinander geschoben werden. Damit hierbei keine Klebstoffkomponente an den Grenzflächen zwischen der ersten und zweiten Kammer 2, 3 austreten kann, ist an der Ringwulst außenseitig ein umlaufendes Dichtelement 10 vorgesehen, welches mit beiden der Längsflächen des Ringspaltes 4 dichtend zusammenwirkt.

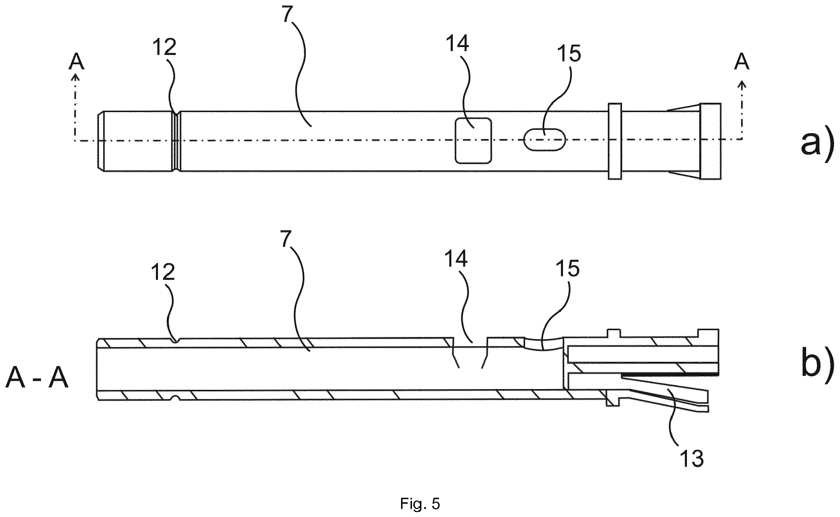

Um ein vorzeitiges und unbeabsichtigtes Vermischen der beiden Klebstoffkomponenten zu verhindern, verschließt das Mischrohr 7 in einer ersten Position das Innere des Mischrohres 7 gegenüber dem Zuführungskanal 6 und dem Ringspalt 4 gibt diese Öffnungen erst in einer zweiten Position frei, wobei die zweite Position durch das vollständig ausgezogene Mischrohr 7 definiert ist. Dies ist insbesondere aus den Detailabbildungen des Mischrohres 7 in den Fign. 5a) und b) zu erkennen. Zu diesem Zweck besitzt das Mischrohr 7 an seinem nach innen weisenden Ende auf der dem Zuführungskanal zugewandten Seite zwei Öffnungen 14, 15 auf, durch die der Zuführungskanal 6 und der Ringspalt 4 mit dem Inneren des Mischrohres 7 verbunden werden können wenn das Mischrohr 7 in die zweite Position gebracht wird. Damit die Öffnungen 14, 15 auch beim Herausziehen des Mischrohres 7 in der korrekten Position verbleiben ist das Mischrohr 7 verdrehsicher in der Mischkammer 5 geführt.

In den Fign. 5a) und b) ist ferner dargestellt, dass das Mischrohr 7 über eine Rückstellsperre 13 verfügt, welche derart ausgebildet ist, dass sie das Mischrohr 7 nach dem Verschieben in die zweite Position fixiert und ein Zurückschieben verhindert, wobei die Rückstellsperre 13 vorliegend als Rastelement in Form eines Widerhakens ausgebildet ist, der sich nach dem Herausziehen des Mischrohres 7 von diesem abstellt. Im Bereich des vorderen Endes des Mischrohres 7 ist ferner eine Rasteinrichtung 12 in Form einer Ringnut eingelassen, die mit einem korrespondierenden Zapfen am Austritt der Mischkammer 5 das Mischrohr 7 im eingeschobenen Zustand gegen unbeabsichtigtes herausrutschen sichert.

In der Mischkammer 5 sind zwischen der Einmündung des Ringspalts 4 und der Außenöffnung der Mischkammer 5 drei Ringspaltdichtungen 17, 18, 19 vorgesehen. Die äußere Ringspaltdichtung 17 dient der Abdichtung zwischen der Mischkammer 5 und der äußeren Oberfläche des Mischrohrs 7 in Position 1 und Position 2. Die mittlere Ringspaltdichtung 18 ist zwischen dem Durchlass des Ringspalts 4 und dem Zuführungskanal 6 angeordnet, um ein unbeabsichtigtes Vermischen der zwei Klebstoff-Komponenten außerhalb des Mischrohres 7 in der Mischkammer 5 zu verhindern. Die innen liegende Ringspaltdichtung 19 ist auf der dem Durchlass des Ringspalts 4 abgewandten Seite des Zuführungskanals 6 angeordnet. Durch die Ringspaltdichtung 19 kann eine Leckage der im Hohlzylinder 2 enthaltenen Komponente in die Mischkammer 5 unterbunden werden.

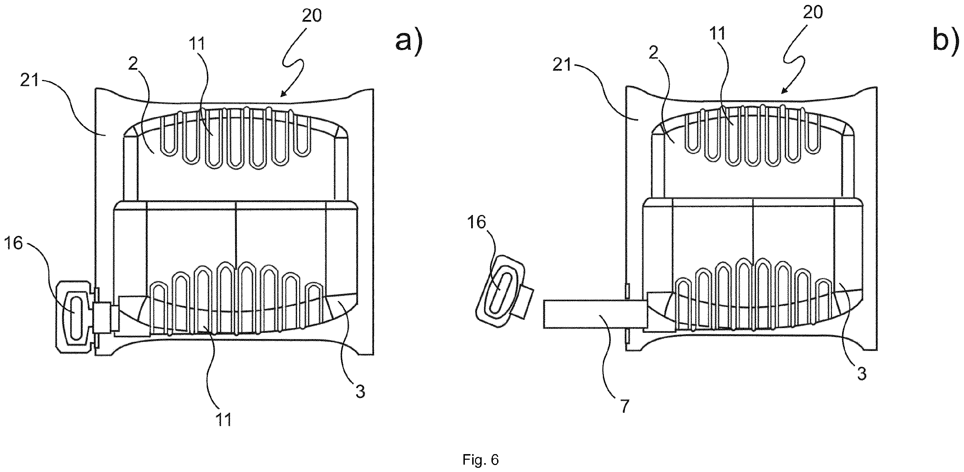

In den Fign. 6a) und b) ist ein erfindungsgemäßes Kit 20 aus einem Dosiersystem 1, wie in den Fign. 1 bis 5 gezeigt, dargestellt. Der Hohlzylinder H und der Vollzylinder V sind auf ihrer jeweiligen Außenseite mit einer in Längsrichtung verlaufender Profilierung 11 in Form einer Riffelung versehen, welche die Griffigkeit und auch die Stabilität erhöhen. In den Kammern 2, 3 sind die zwei Komponenten eines Polyurethan-Harnstoff-Gewebekleber-Systems eingefüllt. Das isocyanatfunktionelle Präpolymer auf Basis von aliphatischen Polyisocyanaten und aminofunktionellen Asparaginsäureestern ist in den Ringspalt 4 der zweiten Kammer 3 und das Polyol in die erste Kammer 2 eingefüllt. Hierzu werden als 2K-Gewebekleber-System solche eingesetzt, wie sie in EP 2 173 782 B1, EP 2 182 018 B1, WO 2011/006606 A1, WO 2010/006714 A2,A3, EP 2 699 615 B1, WO 2013/104563 A1, WO 2012/107375 A1, WO 2013/092506 A1, EP 2 699 614 B1 oder WO 2013/092504 A1 offenbart sind.

Damit kein Sauerstoff, keine Feuchtigkeit oder andere Umwelteinflüsse die Klebstoffkomponenten nachteilig verändern können, ist das Dosiersystem 1 in einen Folienbeutel 21 aus aluminiumbedampfter PET-Folie eingeschweißt und das äußere Ende des Mischrohres 7 mit einem abnehmbaren Verschluss 16 versehen. Der abnehmbare Verschluss 16 dient ferner dazu, das Mischrohr 7 beim Gebrauch aus der Mischkammer 5 herauszuziehen. Zur einfacheren Handhabung ist der Folienbeutel 21 im Bereich des herausragenden Mischrohres 7 mit einer Schwächungslinie versehen, welche ein gezieltes Öffnen des Folienbeutels 21 am Mischrohr 7 erlaubt. So muss bei der Verwendung des Systems nicht der ganze Folienbeutel 21 entfernt werden, es genügt vielmehr, diesem im Bereich des Verschlusses 16 zu öffnen.

Zum Befüllen des erfindungsgemäßen Kits 20 mit einem 2K-Gewebekleber-System kann so vorgegangen werden, dass unter einer trocknen Schutzgasatmosphäre zunächst die erste Kammer 2 mit der einer Komponente des Gewebekleber-Systems befüllt und mit dem den Ringspalt 4 tragende Element 10a in der Weise zusammengesetzt wird, dass der umlaufende Randwulst der ersten Kammer 2 in den Ringspalt 4 eingeführt wird. Anschließend wird das ovale ringförmige zweite Element 10b über die erste Kammer 2 gestreift und mit dem Element 10a über die vorbeschriebene Rasteinrichtung verbunden, wodurch gleichzeitig die erste Kammer 2 verklemmt wird, sodass sie sich nicht mehr lösen kann. Anschließend wird der Ringspalt 4 der zweiten Kammer 3 mit der anderen Komponente des Gewebekleber-Systems befüllt. Dies erfolgt zweckmäßigerweise vor dem Einführen des Mischrohres 7 über die für das Mischrohr 7 vorgesehene Öffnung der zweiten Kammer 3. Alternativ kann auch der Bodenbereich der Kammer 3 unterhalb der Mischkammer 5 abnehmbar ausgeführt sein, wodurch das Einfüllen in den Ringspalt 4 erleichtert wird. Anschließend wird das Mischrohr 7 in das befüllte Dosiersystem 1 bis über die Rasteinrichtung 12 eingesteckt und unter trocknem Schutzgas in einen innenseitig mit Aluminium bedampften Folienbeutel 21 eingeschweißt.

Zur Verwendung des erfindungsgemäßen Kits 20 zum Verschließen einer Wunde, beispielsweise einer Schnittwunde am Arm, wird durch Ziehen am Verschlussstopfen 16 gleichzeitig der Folienbeutel 21 an der Schwächungslinie aufgerissen, das Mischrohr 7 bis zum Erreichen der Endposition herausgezogen und durch leicht kippende Zugbewegung der Verschlussstopfen 16 anschließend abgezogen. Dies kann beispielsweise auch unter Zuhilfenahme der Zähne erfolgen, wenn die Verletzung am Arm ein beidhändiges nicht ermöglicht. Anschließend wird das Dosiersystem mit einer Hand so zusammengedrückt, dass die erste und zweite Kammer 2, 3 ineinandergeschoben werden, wodurch beide Komponenten in das Mischrohr 7 befördert, dort vermischt und an der Öffnung des Mischrohres 7 ausgetragen werden. Die ersten 2 cm austretende Mischung können verworfen werden. Der folgende Klebstoffmischungsstrang wird dann unmittelbar auf die blutende Wunde aufgetragen, wo der Wundklebstoff, beschleunigt durch den Feuchtigkeitskontakt mit der Wunde, je nach Klebstoffsystem binnen 30 Sekunden bis 3 Minuten aushärtet und so die Blutung stoppt.

### Bezugszeichenliste:

- 1: Dosiersystem
- 2: Kammer
- 2a: Stirnseite
- 2b: Stirnseite
- 3: Kammer
- 3a: Stirnseite
- 3b: Stirnseite
- 4: Ringspalt
- 5: Mischkammer
- 6: Zuführungskanal
- 7: Mischrohr
- 8: Anschlageinrichtung
- 9: Anschlageinrichtung
- 10: umlaufende Dichtung
- 10a: Element
- 10b: Element
- 11: Profilierung
- 12: Rasteinrichtung
- 13: Rückstellsperre
- 14: Öffnung
- 15: Öffnung
- 16: Verschlussstopfen
- 17: Äußere Ringspaltdichtung
- 18: Mittlere Ringspaltdichtung
- 19: Innere Ringspaltdichtung
- 20: Kit
- 21: Folienbeutel
- H: Hohlzylinder
- V: Vollzylinder
- L: Längsrichtung

## Patentansprüche

1. Dosiersystem (1) mit wenigstens zwei Kammern (2, 3) für ein wenigstens zwei Komponenten umfassendes Gewebekleber-System,
**dadurch gekennzeichnet, dass**
die erste Kammer (2) als Hohlzylinder (H) ausgebildet ist, der an einer Stirnseite (2a) verschlossen und an seiner gegenüberliegenden Stirnseite (2b) offen ausgebildet ist und die zweite Kammer (3) in einem Vollzylinder (V) als Ringspalt (4) ausgebildet ist und sich der Ringspalt (4) von einer dem Hohlzylinder (H) zugewandten Stirnseite (3a) bis zu einer an dem gegenüberliegenden Ende (3b) des Vollzylinders (V) angeordneten Mischkammer (5) erstreckt und der Hohlzylinder (H) auf der Seite seiner offenen Stirnseite (2b) in dem Ringspalt (4) des Vollzylinders (V) in Längsrichtung (L) verschiebbar angeordnet ist,
wobei in dem Vollzylinder (V) wenigstens ein Zuführungskanal (6) ausgebildet ist, der sich innenseitig des Ringspaltes (4) von der dem Hohlzylinder (H) zugewandten Stirnseite (3a) des Vollzylinders (V) bis in die Mischkammer (5) erstreckt, wobei in der Mischkammer (5) ein Mischrohr (7) vorgesehen ist, welches aus dem Vollzylinder (V) seitlich herausragt und im Wesentlichen quer zur Längsausdehnung des Vollzylinders (V) verschiebbar angeordnet ist und das in einer ersten Position das Innere des Mischrohres (7) gegenüber dem Zuführungskanal (6) und dem Ringspalt (4) verschließt und in einer zweiten Position freigibt.

2. Dosiersystem nach Anspruch 1, **dadurch gekennzeichnet, dass** im Bereich der Stoßkante der offenen Stirnseite (2b) des Hohlzylinders (H) eine Anschlageinrichtung (8) ausgebildet ist, die mit einer an der dem Hohlzylinder (H) zugewandten Stirnseite (3a) des Vollzylinders (V) ausgebildeten korrespondierenden Anschlageinrichtung (9) in der Weise zusammenwirkt, dass der Hohlzylinder (H) und der Vollzylinder (V) nur bis zum gegenseitigen Kontakt der Anschlageinrichtungen (8, 9) auseinander gezogen werden können, wobei die Anschlageinrichtung (8) am Hohlzylinder (H) insbesondere als umlaufender Ringwulst ausgebildet ist, der mit einer am Vollzylinder (V) innenseitig umlaufenden Anschlagfläche als korrespondierende Anschlageinrichtung (9) zusammenwirkt und/ oder an der Ringwulst bevorzugt ein umlaufendes Dichtelement (11) vorgesehen ist, welches mit wenigstens einer, vorzugsweise mit beiden, der Längsflächen des Ringspaltes (4) dichtend zusammenwirkt.

3. Dosiersystem nach Anspruch 1 oder 2, **dadurch gekennzeichnet, dass** die Längsausdehnung des Hohlzylinders (H) derart gewählt ist, dass die Höhe der Innenwand weitestgehend identisch mit der Tiefe des Ringspalts (4) ist.

4. Dosiersystem nach einem der vorstehenden Ansprüche, **dadurch gekennzeichnet, dass** das Dosiersystem zwei Kammern (2, 3) aufweist und die Volumina der beiden Kammern (2, 3) in einem Verhältnis 1 : 10 bis 10 : 1 zueinander stehen, bevorzugt 5 : 1 bis 1 : 5 und/ oder das Volumen der ersten Kammer (2) zum Volumen der zweiten Kammer (3) im Verhältnis 5 : 1 bis 3 : 1 beträgt, bevorzugt 4,5 : 1 bis 3,5 : 1.

5. Dosiersystem nach einem der vorstehenden Ansprüche, **dadurch gekennzeichnet, dass** der Hohlzylinder (H) und der Vollzylinder (V) mit einer Sicherungseinrichtung versehen sind, die den Hohlzylinder H) und den Vollzylinder (V) gegen ein unbeabsichtigtes gegeneinander Verschieben sichert.

6. Dosiersystem nach einem der vorstehenden Ansprüche, **dadurch gekennzeichnet, dass** innerhalb des Mischrohres (7) Mischelemente angeordnet sind, wobei das Mischrohr (7) insbesondere als Statikmischer ausgebildet ist.

7. Dosiersystem nach einem der vorstehenden Ansprüche, **dadurch gekennzeichnet, dass** an dem Mischrohr (7) eine Rasteinrichtung (12) vorgesehen ist, die das Mischrohr (7) in der ersten Position gegen unbeabsichtigtes Herausziehen des Mischrohrs (7) fixiert.

8. Dosiersystem nach einem der vorstehenden Ansprüche, **dadurch gekennzeichnet, dass** an dem Mischrohr (7) eine Rückstellsperre (13) vorgesehen ist, welche derart ausgebildet ist, dass sie das Mischrohr (7) nach dem Verschieben in die zweite Position fixiert und ein Zurückschieben verhindert, wobei die Rückstellsperre (13) bevorzugt als Flügel oder Widerhaken ausgebildet ist.

9. Dosiersystem nach einem der vorstehenden Ansprüche, **dadurch gekennzeichnet, dass** das Mischrohr (7) verdrehsicher in der Mischkammer (5) geführt und an seinem nach innen weisenden Ende auf der dem Zuführungskanal zugewandten Seite mit einer oder mehreren Öffnungen (14, 15) versehen ist, durch die der Zuführungskanal (6) und / oder der Ringspalt (4) mit dem Inneren des Mischrohres (7) verbunden werden kann wenn das Mischrohr (7) in die zweite Position gebracht wird.

10. Dosiersystem nach einem der vorstehenden Ansprüche, **dadurch gekennzeichnet, dass** das Mischrohr (7) auf seiner aus dem Vollzylinder (V) herausragenden Seite mit einem abnehmbaren Verschluss (16) versehen ist.

11. Dosiersystem nach einem der vorstehenden Ansprüche, **dadurch gekennzeichnet, dass** die Mischkammer (5) innerhalb des Vollzylinders (V) ausgebildet ist.

12. Dosiersystem nach einem der vorstehenden Ansprüche, **dadurch gekennzeichnet, dass** in der Mischkammer (5) zwischen der Einmündung des Ringspalts und der Außenöffnung der Mischkammer eine Ringspaltdichtung (17) vorgesehen ist, die der Abdichtung zwischen der Mischkammer (5) und der äußeren Oberfläche des Mischrohrs (7) in Position 1 und Position 2 dient.

13. Dosiersystem nach einem der vorstehenden Ansprüche, **dadurch gekennzeichnet, dass** der Zuführungskanal (6) im Wesentlichen parallel zum Ringspalt (4) verläuft und von diesem insbesondere wenigstens 2 mm beabstandet ist, vorzugsweise wenigstens 3 mm.

14. Dosiersystem nach einem der vorstehenden Ansprüche, **dadurch gekennzeichnet, dass** Hohlzylinder (H) und Vollzylinder (V) gemeinsam in einen Beutel (21) eingeschlossen sind, wobei der Beutel (21) vorzugsweise im Bereich des herausragenden Mischrohres (7) mit einer Schwächungslinie versehen ist, welche ein gezieltes Öffnen des Beutels (19) am Mischrohr (7) erlaubt.

15. Kit (20) aus einem Dosiersystem (1) nach einem der Ansprüche 1 bis 14, und einem darin befindlichen zwei Komponenten umfassenden Gewebekleber-System, sowie optional eine Verfahrensanleitung zum Verschließen einer Wunde mithilfe des Kits, wobei das Gewebekleber-System bevorzugt wenigstens einen Asparaginsäureester als Härter mit optional wenigstens einem Füllstoff als erste Komponente und als zweite Komponente ein isocynatfunktionelles Präpolymer umfasst, wobei sich die erste Komponente besonders bevorzugt in der ersten Kammer (2) und die zweite Komponente bevorzugt in der zweiten Kammer (3) des Dosiersystems befindet.

16. Verfahren zum Verschließen einer Wunde mithilfe eines Kits gemäß Anspruch 15 umfassend die Schritte:
a) Herausziehen des Mischrohrs (7) bis zum Erreichen der zweiten Position;
b) Druckausübung auf den Hohlzylinder (H) und den Vollzylinder (V) relativ zueinander in Längsrichtung der Zylinder (H, V), wodurch die beiden Komponenten des Gewebekleber-Systems in Richtung der Mischkammer (5) gefördert und beim Durchfließen des Mischrohres (7) miteinander vermischt werden;
c) Optionales Verwerfen der wenigstens ersten 10 mm aus dem Mischrohr (7) austretenden Gewebeklebers;
d) Auftragen des unter weiterer Druckausübung aus dem Mischrohr (7) austretenden Gewebeklebers auf die Wunde;
e) Aushärtenlassen des Gewebeklebers.
